# DEMANDE DE BREVET EUROPEEN

(11) **EP 0 714 664 A1**
(43) Date de publication de la demande: **05.06.1996**
(21) Numéro de dépôt: 95420331.1
(22) Date de dépôt: 27.11.1995
(51) Int. Cl.: A61L 2/08, A61L 2/20

(54) **Cellule à rayonnement gamma pour la stérilisation de produits industriels**

(30) Priorité: 30.11.1994 FR 9414611
(71) Demandeur: Lanotte, Michel, F-69330 Jons (FR)
(72) Inventeur: Lanotte, Michel, F-69330 Jons (FR)
(74) Mandataire: Monnier, Guy

(57) **Abrégé**

La cellule à rayonnement Gamma comprend un convoyeur aérien (6) pour le déplacement de nacelles (7-7a) supportant les produits à traiter. Le plancher (3) comporte un puits central (26) permettant le stockage d'une source d'irradiation (27) qui peut être manoeuvrée verticalement. Des canalisations (39 et 40) relient ce puits (26) à une réserve d'oxygène et à un réservoir pour le stockage de l'ozone produit par radiolyse, cet ozone pouvant être admis dans le caisson (7a) des nacelles (7).

## Description

La présente invention a pour objet une cellule de stérilisation pour produits industriels qui tire parti des effets, bien connus en eux-mêmes, de l'énergie du rayonnement GAMMA sur les liaisons interatomiques des chaînes A.R.N. et A.D.N., en créant ainsi les conditions de stérilité désirées sur les populations microbiennes ou microbiologiques.

Si une telle cellule est susceptible d'être utilisée pour le traitement de tous produits industriels quelle que soit leur nature, les études et les essais effectués par le Demandeur ont révélé un intérêt tout particulier pour la stérilisation de divers produits médicaux, notamment en milieu hospitalier.

La cellule suivant l'invention, du genre constitué par une enceinte entièrement blindée pourvue d'au moins une porte étanche d'accès elle-même blindée, est caractérisée en ce qu'elle comprend en combinaison :
- un puits central ménagé dans son plancher afin de former stockage sec pour une source d'irradiation ;
- un mécanisme de levage pour la manoeuvre verticale de la source d'irradiation qui peut ainsi affecter une position haute de travail et une position basse de stockage ;
- un circuit pour la production d'ozone par radiolyse d'un courant d'oxygène, associé au puits central, lequel circuit, mis en oeuvre lorsque ladite source est à la position basse de stockage, relie ce puits à une réserve d'oxygène et à un réservoir de stockage d'ozone ;
- un carrousel constitué par une série de nacelles destinées à recevoir les produits à stériliser et par un convoyeur propre à déplacer lesdites nacelles à l'intérieur de la cellule ;
- des moyens de connexion pour relier l'espace intérieur de l'enceinte ou caisson des nacelles au réservoir de stockage d'ozone et à un système d'évacuation ;
- et des moyens d'insufflation et d'aspiration pour le traitement adéquat de l'atmosphère intérieure de la cellule.

On conçoit qu'une telle cellule, à fonctionnement entièrement autonome, permet d'adapter de manière précise le traitement de stérilisation à la nature exacte des produits concernés.

Le dessin annexé, donné à titre d'exemple, permettra de mieux comprendre l'invention, les caractéristiques qu'elle présente et les avantages qu'elle est susceptible de procurer :
Fig. 1 est une coupe horizontale montrant très schématiquement l'agencement général d'une cellule de stérilisation établie conformément à l'invention
Fig. 2 est la coupe verticale correspondante.
Fig. 3 est une coupe verticale à plus grande échelle illustrant la suspension et l'entraînement de l'une des nacelles du carrousel.
Fig. 4 est un schéma du circuit pour la production d'ozone et pour le remplissage des nacelles.

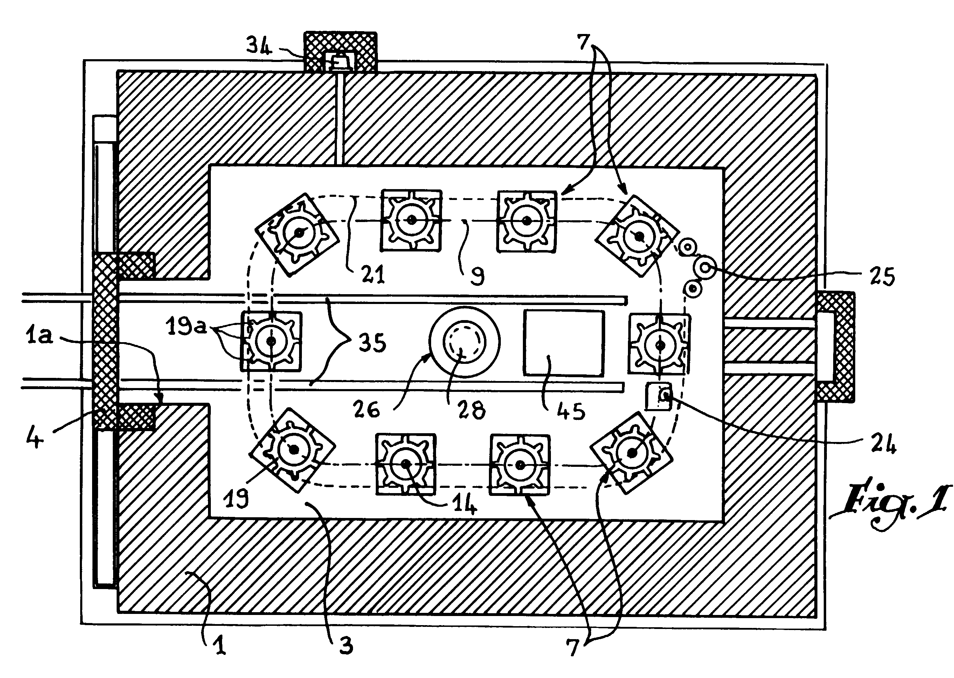

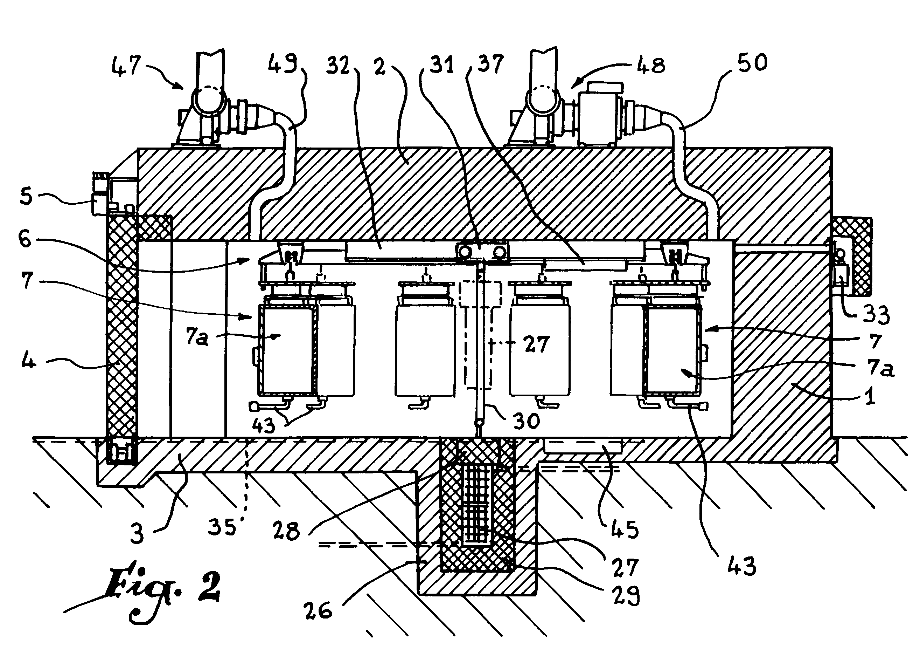

La cellule représentée en fig. 1 et 2 comprend essentiellement une enceinte formée par une paroi latérale 1 à section rectangulaire, un toit 2 et un plancher 3, ces trois éléments 1, 2 et 3 étant constitués par des blindages propres à assurer une protection biologique efficace de l'environnement. La paroi latérale 1 est pourvue d'une ouverture la équipée d'une porte blindée étanche 4 déplacée à l'aide d'un mécanisme moteur 5.

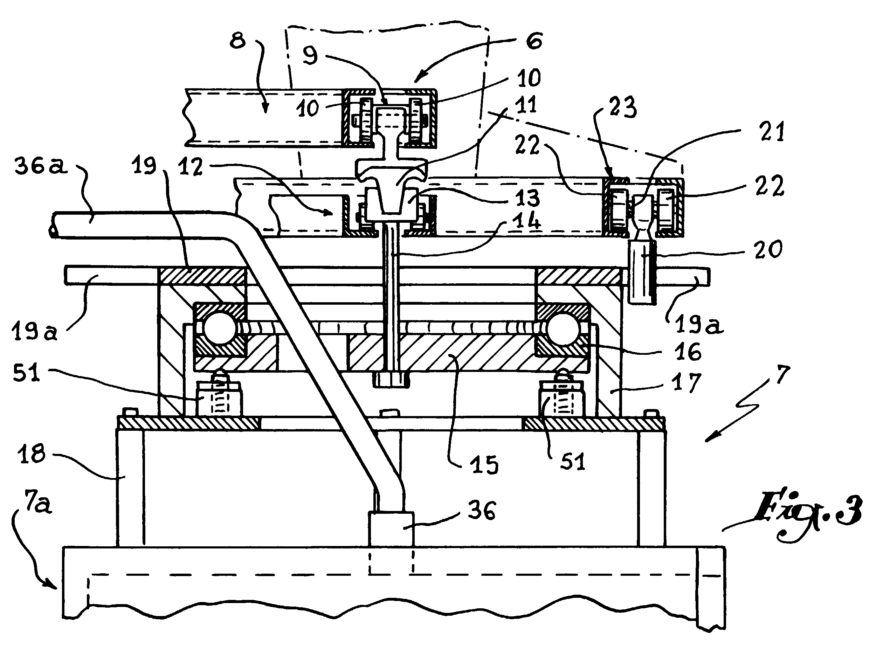

Le toit 2 supporte un carrousel qui comprend un convoyeur aérien 6 pour la suspension et le déplacement de nacelles 7, au nombre de dix dans l'exemple de réalisation considéré. Comme montré en fig. 3, le convoyeur 6 comprend lui-même un rail supérieur 8 à l'intérieur duquel se déplace une chaîne 9 supportée de place en place par des galets de roulement 10 ; au niveau de chaque paire de galets 10, la chaîne 9 est pourvue d'un taquet d'entraînement 11 tourné vers le bas.

Au-dessous du rail 8 et établi au même profil en plan que celui-ci, est prévu un rail inférieur 12 à l'intérieur duquel circulent dix chariots 13, chacun de ceux-ci étant déplacé à l'aide de l'un des taquets 11 de la chaîne 9. Chaque chariot 13 est solidaire d'un axe vertical 14 tourné vers le bas pour supporter l'une des nacelles 7.

A cet effet, la base de chaque axe 14 est rigidement solidaire d'un plateau fixe 15 qui, par l'intermédiaire d'un roulement à billes 16, supporte une douille tournante 17 rendue solidaire de la partie supérieure de l'une des nacelles 7 par une cage 18.

Il convient d'observer que contre le bord supérieur de la douille 17 est fixé un disque 19 dont la périphérie, prévue débordante par rapport à ladite douille, est découpée d'encoches radiales qui définissent des doigts 19a, au nombre de huit dans l'exemple de réalisation envisagé. Comme on l'exposera plus loin, ces doigts 19a sont aptes à coopérer avec des taquets 20 portés par une chaîne 21, laquelle est supportée de place en place par des galets 22 circulant dans un rail fixe ou gaine 23. Cette dernière s'étend parallèlement aux rails superposés 8 et 12, en étant disposée vers l'extérieur par rapport à ceux-ci.

La chaîne 9 renfermée par le rail supérieur 8 est entraînée en translation par un moto-réducteur 24 (fig. 1), tandis que la chaîne 21 montée dans la gaine latérale 23 coopère, pour son entraînement, avec un moto-réducteur indépendant 25. Les deux moto-réducteurs 24 et 25 sont fixés contre le toit 2.

Dans la partie centrale du plancher 3 de la cellule est ménagé un puits 26. A l'intérieur de ce puits 26 est logée une source d'irradiation 27 constituée, à la façon en soi connue, par un panier à l'intérieur duquel sont montés concentriquement des crayons en cobalt radioactif (cobalt 60) ou autre source de rayonnements GAMMA. Le panier de la source 27 est suspendu à un bouchon 28 (fig. 2) formant couvercle pour la cuve interne 29 du puits central 26, l'ensemble étant ainsi propre à définir un stockage sec.

Au bouchon ou couvercle 28 est associé un mouflage dont le câble 30 est renvoyé sur un chariot 31 qui circule sur un rail supérieur 32 fixé contre le toit 2 de la cellule. Ce câble 30 est commandé par un treuil 33 fixé latéralement à l'extérieur de la paroi 1, tandis que le chariot 31 peut être déplacé horizontalement le long du rail 32 par un treuil 34. Des moyens appropriés (non représentés) sont prévus pour assurer le guidage de l'ensemble 27-28 lors de ses déplacements verticaux.

Sur le plancher 3 de la cellule sont fixés des rails 35 pour le déplacement d'un chariot auto-moteur (non représenté) qui porte un robot manipulateur destiné à permettre, en liaison avec les treuils 33 et 34, la mise en place initiale de la source d'irradiation 27 et son remplacement lorsqu'elle est épuisée.

La partie supérieure de certaines au moins des nacelles 7 est pourvue d'un joint tournant 36 (fig. 3) qui débouche dans un caisson 7a fixé à la cage 18 des nacelles envisagées et sur lequel est assujettie une canalisation radiale 36a dont l'extrémité libre, pourvue d'un raccord à clapet, est susceptible d'être branchée sur l'une des canalisations flexibles d'un dispositif de connexion 37. Ce dernier, fixé contre le toit 2 de la cellule, est relié à l'atmosphère à travers des filtres appropriés.

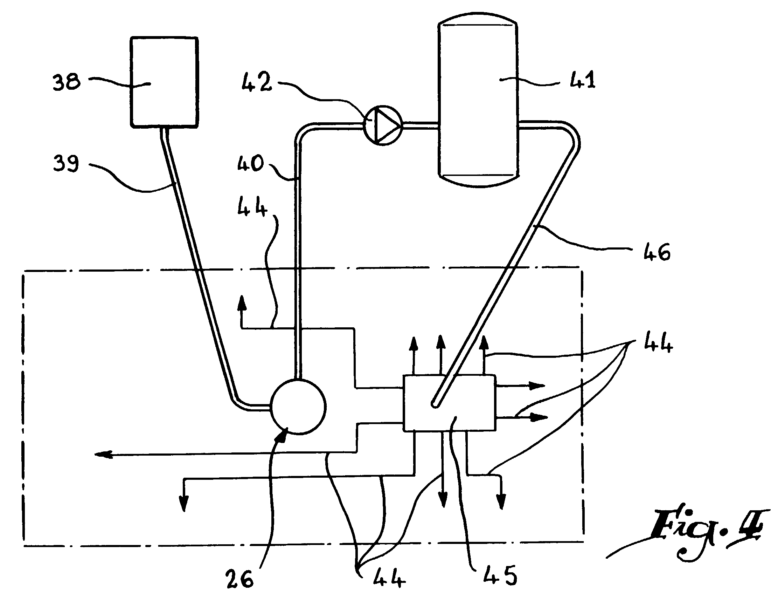

La cellule est dotée d'un circuit de production d'ozone par radiolyse qui comprend une source d'oxygène 38 (fig. 4) mise en communication par une canalisation 39 avec la base de la cuve 29 du puits central 26 lorsque la source d'irradiation 27 est en position basse de stockage à l'intérieur de la cuve 29. Cette dernière est reliée par une canalisation supérieure 40 à un réservoir de stockage d'ozone 41 à travers un compresseur 42.

La base du caisson 7a des nacelles 7 comporte en son centre un joint tournant associé à une canalisation radiale 43 (fig. 2). Chaque canalisation 43 peut être reliée par une canalisation flexible (représentée sur le schéma de fig. 4 sous la forme de simples flèches référencées 44) à un dispositif de connexion 45 logé dans le plancher 3 de la cellule ; ce dispositif 45 communique par une canalisation 46 avec le réservoir de stockage 41.

La cellule suivant l'invention est encore équipée de deux groupes moto-ventilateurs 47 et 48 fixés sur le toit 2 et équipés de systèmes de filtration adéquats. Le groupe 47 aspire par un conduit 49 l'air ou gaz qui est insufflé dans l'atmosphère de la cellule par un conduit 50 relié à une source appropriée à travers le groupe 48 ; c'est sur ce conduit d'extraction 49 qu'aboutit la liaison prévue entre le dispositif de connexion 37 et l'atmosphère. Les groupes 47 et 48 sont asservis de façon à ce qu'une légère dépression règne dans la cellule.

Il va de soi que le caisson 7a de chaque nacelle 7 est équipé d'une porte assurant une fermeture hermétique ; ce caisson 7a comporte des supports et autres agencements aptes à permettre la suspension et le rangement rationnels des produits à stériliser. Suivant les cas, les opérations de chargement et de déchargement des nacelles 7 peuvent être effectuées soit à l'intérieur de la cellule, soit à l'extérieur de celle-ci, le convoyeur 6 étant alors muni d'aiguillages appropriés.

Quel que soit le mode de chargement, on conçoit qu'une fois les produits à stériliser installés dans le caisson 7a des nacelles 7, lesdits produits sont susceptibles de recevoir le traitement qui convient le mieux à leur nature et à leur état, du fait de la souplesse de fonctionnement remarquable de la cellule suivant la présente invention. On observera en particulier :
- qu'à l'aide des groupes moto-ventilateurs 47 et 48 et des conduits 49 et 50, l'espace intérieur de l'enceinte 1-2-3 peut être empli d'un gaz apte à favoriser la stérilisation (qui peut d'ailleurs être de l'ozone directement prélevé dans le puits 26), les extrémités libres des canalisations 36a et 43 étant bien entendu maintenues ouvertes, ainsi que les portes des caissons 7a ;
- qu'au contraire l'espace intérieur du caisson 7a de chaque nacelle 7 peut être empli d'ozone ou autre gaz à travers les canalisations 43 et 36a sus-mentionnées, et ce par le moyen des dispositifs de connexion 45 et 37 ;
- qu'au moyen du treuil 33, la source 27 peut être extraite du puits central 26 pour l'irradiation du contenu des caissons des nacelles 7, ce qui a évidemment pour effet de faire cesser la production d'ozone à l'intérieur de la cuve 29.
- que par mise en fonctionnement du seul moto-réducteur 25, les nacelles 7 peuvent être entraînées en rotation suivant leur axesupport 14 tout en étant immobiles en translation, les taquets 20 portés par la chaîne 21 coopérant avec les doigts 19a du disque 19 pour mettre en rotation chaque nacelle ;
- que moyennant mise en fonctionnement du seul moto-réducteur 24, il est possible de déplacer en translation l'ensemble des nacelles 7 dans la cellule, en les faisant tourner sur elles-mêmes par suite de l'immobilisation de la chaîne latérale 21 et des taquets 20 ;
- et que dans le cas où les deux moto-réducteurs 24 et 25 sont mis en fonctionnement, les nacelles 7 se déplacent en translation sans tourner sur elles-mêmes.

On notera que lorsque les nacelles 7 sont entraînées en rotation suivant leur axe, il est avantageux de leur conférer un mouvement pas à pas parfaitement régulier. Ce résultat peut être obtenu à l'aide de positionneurs élastiques à bille 51 (fig. 3) portés par la cage 18 et propres à coopérer avec des empreintes ménagées dans la face inférieure du plateau fixe 15 correspondant.

Il convient d'observer qu'on peut disposer à l'intérieur de la cellule des rampes ou buses agencées pour permettre l'admission d'un courant d'oxygène au cours du traitement (source 27 en position haute de travail) pour être récupéré sous forme d'ozone soit en vue de son stockage, soit pour une autre utilisation extérieure en direct. On conçoit par ailleurs que l'entraînement en rotation des nacelles 7 suivant leur axe est susceptible d'être obtenu à l'aide de moteurs indépendants, du type pas à pas.

On comprend également que la manoeuvre verticale de la source d'iradiation 27 peut être opérée à l'aide d'un vérin pneumatique ou hydraulique faisant office de mât central, aux lieu et place du système de câble de levage 30-33 ci-dessus exposé.

Le puits central 26 et la cuve 29 qu'il renferme sont susceptibles d'être prévus de manière démontable afin de pouvoir être manipulés et utilisés à la manière d'un emballage pour le transport de la source.

## Revendications

1. Cellule à rayonnement GAMMA pour la stérilisation de produits industriels, du genre constitué par une enceinte blindée (1-2-3) pourvue d'au moins une porte étanche d'accès (4), caractérisée en ce qu'elle comprend en combinaison :
- un puits central (26) ménagé dans le plancher (3) afin de former stockage sec pour une source d'irradiation (27) ;
- un mécanisme de levage (30-31-32-33) pour la manoeuvre verticale de la source (27) qui peut ainsi affecter une position haute d'irradiation et une position basse de stockage ;
- un circuit pour la production d'ozone par radiolyse d'un courant d'oxygène, associé au puits (26), lequel circuit, mis en oeuvre lorsque la source (27) est à la position basse de stockage, relie le puits précité à une réserve d'oxygène (38) et à un réservoir de stockage d'ozone (41) ;
- un carrousel constitué par une série de nacelles (7) destinées à supporter les produits à stériliser et par un convoyeur (6) propre à déplacer lesdites nacelles (7) à l'intérieur de l'enceinte (1-2-3) ;
- des moyens de connexion (45, 37) pour relier l'espace intérieur de rangement de chaque nacelle (7) au réservoir de stockage d'ozone (41) et à un système d'évacuation ;
- et des moyens d'insufflation (48) et d'aspiration (47) pour le traitement adéquat de l'atmosphère intérieure de l'enceinte (1-2-3).

2. Cellule suivant la revendication 1, caractérisée en ce que chaque nacelle (7) est montée à rotation suivant un axe vertical (14) qui la relie au convoyeur (6), en vue d'être déplacée angulairement lorsque désiré.

3. Cellule suivant la revendication 2, caractérisée en ce qu'un roulement (16) est interposé entre la partie supérieure (17-18) de chaque nacelle (7) et un plateau fixe (15) porté par l'axe (14), tandis qu'un disque encoché (19) solidaire de ladite partie (17-18) coopère avec des taquets (20) portés par une chaîne (21) disposée parallèlement au convoyeur (6).

4. Cellule suivant la revendication 3, caractérisée en ce que la chaîne (21) est entraînée par un moto-réducteur (25) indépendant de celui (24) qui assure l'entraînement du convoyeur (6).

5. Cellule suivant l'une quelconque des revendications 2, 3 et 4, caractérisée en ce que certaines au moins des nacelles (7) sont pourvues d'un caisson étanche (7a) dont l'espace intérieur peut être mis en communication par des joints tournants (36) avec les dispositifs de connexion (37, 45).

6. Cellule suivant l'une quelconque des revendications 1 à 5, caractérisée en ce que les moyens pour le traitement de l'atmosphère de l'enceinte (1-2-3) comprennent deux groupes moto-ventilateurs (47, 48) qui sont asservis entre eux de manière à engendrer une légère dépression à l'intérieur de ladite enceinte.
